# FASCICULE DE BREVET EUROPEEN

(11) **EP 0 646 370 B1**
(45) Date de publication et mention de la délivrance du brevet: **24.07.1996**
(21) Numéro de dépôt: 94402182.3
(22) Date de dépôt: 29.09.1994
(51) Int. Cl.: A61K 7/48

(54) **Compositions cosmétiques et dermatologiques associant céramides et acide linoléique, leur préparation**
Kosmetische und dermatologische Zusammensetzungen, die eine Kombination aus Ceramiden und Linolsäure enthalten
Cosmetic and dermatological compositions containing an association of ceramides and linoleic acids, their preparation

(30) Priorité: 30.09.1993 FR 9311641
(43) Date de publication de la demande: 05.04.1995
(73) Titulaire: ROUSSEL UCLAF, F-93230 Romainville (FR)
(72) Inventeur: Enjolras, Odile, F-92190 Meudon (FR)
(74) Mandataire: Jacobi, Markus Alexander

(56) Documents cités:
- EP-A- 0 556 957
- WO-A-90/01323
- HAPPI (HOUSEHOLD & PERSONAL PRODUCTS INDUSTRY), vol.27, no.12, 1990, RAMSEY, NJ, USA page 2216 'skin care ingredient'

## Description

La présente invention concerne de nouvelles compositions cosmétiques ou dermatologiques associant céramides et une source d'acide linoléique. Dans WO-A 90/01323 et EP-A 0556957 des compositions pour le traitement de la peau sont décrites qui contiennent un acide gras, un stéroide et éventuellement un céramide.

L'eau représente 70 % du poids du corps humain. En ce qui concerne la peau, le derme représente 15 à 18 % de cette eau. Sa teneur est de l'ordre de 70 %. La teneur en eau de l'épiderme varie considérablement avec la profondeur et le type de couche considéré. La couche de Malpighi (assises cellulaires de l'épiderme au-dessus de la couche basale) contient elle aussi 70 % d'eau. Quant à la couche cornée (ou Stratum Corneum), elle en contient 10 à 13 %.

La couche cornée normale (orthokératosique), d'un épiderme normal, est un pavage de cellules dépourvues de noyaux qu'il est commode d'illustrer selon le modèle d'Elias : un mur de briques, les cornéocytes, briques protéiques bourrées de kératine englobée dans l'enveloppe protéique cornée (enveloppe cornée faite de plusieurs protéines : kératolinine, involucrine, loricrine...), briques scellées par un ciment lipidique inter-cellulaire, constitué d'archéocouches de lipides lamellaires, les céramides.

Le pouvoir hygroscopique des cornéocytes et leur hydrophilie sont liés à l'existence des NMF (ou Natural Moisturizing Factor, facteurs naturels d'hydratation). Ces facteurs d'hydratation cornéocytaire proviennent de la désintégration par protéolyse de protéines épidermiques riches en histidine stockées dans les grains de kératohyaline (filaggrine --> NMF). Les NMF sont riches en acides aminés, acide lactique, acide pyrrolidone carboxylique, urée... Ces substances captent l'eau du milieu extérieur vers l'intérieur des cornéocytes.

Les céramides, sphingolipides de base, sont synthétisées dans les corps d'Odland et déversées dans les espaces extracellulaires à la partie haute de la couche granuleuse.

Les céramides sont des lipides en bicouches lamellaires, relativement rigides, alors que les phospholipides des membranes de la couche de Malpighi sont fluides.

Aussi assurent-elles la cohésion entre les cornéocytes, cellules dépourvues de ces structures d'ancrage que sont les desmosomes, au niveau de l'épiderme vivant.

Les céramides créent un réseau inter-cornéocytaire feuilleté, véritable treillis que le microscope électronique peut visualiser après cryofracture du stratum corneum. La structure quasi-cristalline des céramides leur confère une grande stabilité mécanique, une imperméabilité à l'eau et une résistance à l'oxydation au contact de l'air. Le treillis céramidique sertit les cornéocytes et assure à la surface cutanée son étanchéité et sa cohésion.

Les lipides de la barrière cornée contrôlent les pertes d'eau trans-épidermiques : s'ils diminuent, les pertes s'élèvent. Ce flux de perte d'eau trans-épidermique est un flux minimum d'eau non retenue par l'organisme qui en traversant l'épiderme (non vascularisé), lui apporte des nutriments, de l'oxygène si ce flux augmente, la peau devient rugueuse et sèche.

Les lipides de la couche cornée protègent les NMF. Le modèle d'Elias devient un mur de briques protéiques hydrophiles (les cellules cornées) scellées par un ciment lipidique céramidique hydrophobe. Toute altération d'un de ces constituants altèrera cette barrière hydrophile-lipophile de surface et modifiera les échanges d'eau et la desquamation de surface, la peau souffrira de xérose.

On connaît au moins 7 types de céramides dans la peau humaine, qui sont les suivants : 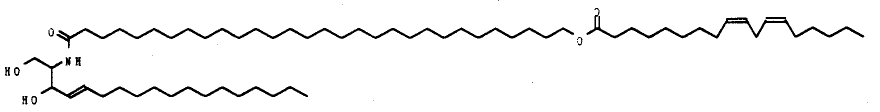
N-(ω-acyloxy)-acylsphingosine 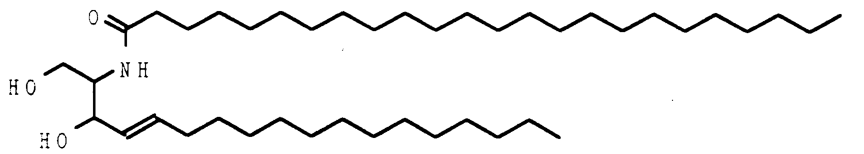
N-acylsphingosine 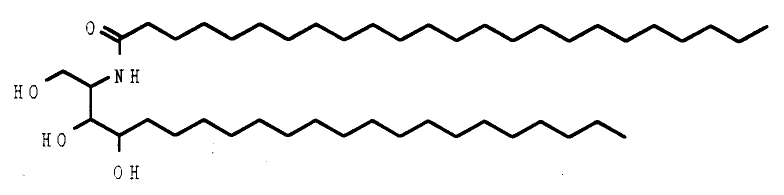
N-acylphytosphingosine 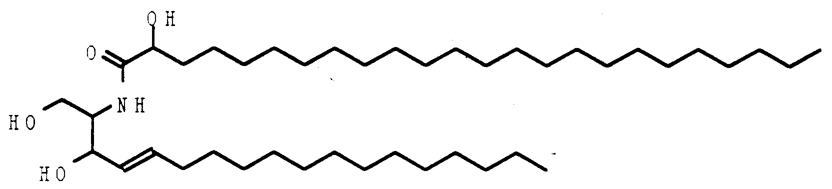
N-(α-hydroxy) acylsphingosine 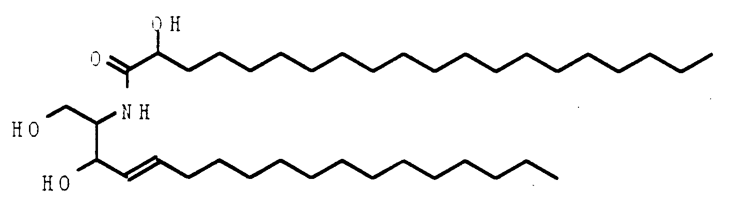
N-(α-hydroxy) acylsphingosine 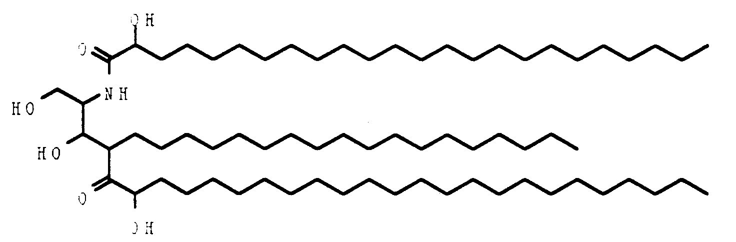
Conformation chimique exacte non définitive 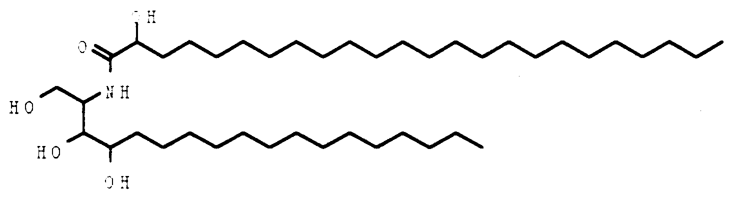
N-(α-hydroxy) acylphytosphingosine
Il existe de nombreux produits sur le marché contenant des céramides. On peut en effet extraire les céramides de cerveaux d'animaux, particulièrement les boeufs, ou bien les extraire de végétaux comme le blé, ou encore les extraire de levures ou bien encore les faire produire par des micro-organismes génétiquement modifiés.

Les céramides sont le plus souvent un mélange de glycosyl-céramides et de céramides véritables. Il existe également des céramides de synthèse dont la structure est identique aux céramides naturelles; il existe également des molécules de synthèse comme la céramide HO3 de la Société SEDERMA dont la structure est proche de celle des céramides naturelles.

De nombreux produits cosmétiques lancés sur le marché contiennent des céramides. Celles-ci sont très variées et proviennent des différentes sources citées ci-dessus. Il a été montré notamment qu'une crème contenant des glycosyl-céramides d'origine bovine ou de la céramide HO3 permettait la restauration de la fonction barrière.

Cependant, s'il est bon de remplacer les céramides naturelles sur une peau sèche qui en manque, il faut s'interroger sur l'origine du déficit en céramides de cette peau sèche ou très sèche. Il peut s'agir d'une délipidation par excès de lavage, nombreux bains, transpiration-essuyage, usage trop fréquent de démaquillants non appropriés. Dans tous les cas, les corps d'Odland qui synthétisent les céramides devraient agir et déverser dans le stratum corneum ces feuillets lipidiques organisés. Mais le stress des kératinocytes (chaleur, soleil, agressions chimiques ou bactériologiques) et le vieillissement peuvent diminuer la vitesse de réparation.

D'autre part, de nombreux essais ont été réalisés tant en dermatologie qu'en cosmétique, en incorporant des huiles riches en acide linoléique pour traitement par voix topique des peaux sèches et très sèches, comme par exemple les peaux xérosiques, ichtyosiques, atopiques, psoriasiques comme l'a montré J.J. GUILHOU (Montpellier) dans une revue récente des tests cliniques réalisés (Ann. Dermatol. Venerol., 1992, 119, 233-239) les résultats sont variables parfois positifs, parfois nuls (vis à vis d'un placebo).

L'activité de l'acide linoléique (et des autres acides gras essentiels) est assez bien comprise dans les productions de prostaglandines et leucotriènes. Mais ce n'est pas ce mécanisme qui peut expliquer à lui seul la restauration de la fonction barrière.

Il a été trouvé et c'est l'objet de la présente invention que l'association d'un céramide spécifique et d'une source d'acide linoléique permettait de renforcer le rôle barrière du Stratum Corneum.

La présente invention consiste donc à associer dans un produit de traitement dermatologique ou de soin cosmétique, une céramide et d'une source d'acide linoléique.

La présente invention a ainsi pour objet de nouvelles compositions cosmétiques ou dermatologiques pour les soins de la peau caractérisées en ce qu'elles renferment une céramide et de l'acide linoléique.

L'étude des effets produits par l'utilisation en cosmétique et en dermatologie de telles compositions, objet de l'invention, montre que l'on pallie à la première urgence de la peau sèche ou très sèche, pathologique ou non ; ou apporte un premier substitut immédiat à la fonction barrière par les céramides, et en même temps on apporte à la peau sèche ou très sèche les éléments nécessaires à une reprise du métabolisme.

De telles compositions cosmétiques ou dermatologiques sont donc destinées à renforcer la fonction barrière du stratum corneum des peaux sèches et très sèches. Des résultats remarquables ont ainsi été obtenus par une telle association de céramides et acide linoléique, objet de la présente invention, ainsi que le montrent les résultats décrits ci-après.

La présente invention a donc pour objet des compositions dermatologiques ou cosmétiques telles que définies ci-dessus, caractérisées en ce que l'on utilise une céramide de synthèse, semi-synthétique ou semblable aux céramides naturelles, la céramide HO3 de la Société SEDERMA, dont la formule est :

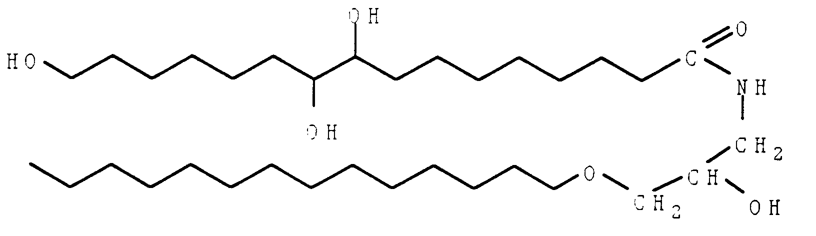

### CERAMIDE HO3

La teneur en céramides des préparations selon l'invention peut varier de 0,01 % à 30 %, préférablement de 0,1 à 15 %, plus préférentiellement de 0,1 à 1%.

La présente invention a plus précisément pour objet les compositions dermatologiques ou cosmétiques telles que définies ci-dessus, caractérisées en ce que la teneur en céramides est de 0,01 % à 30 % et de préférence de 0,1 à 15 %.

La présente invention a ainsi pour objet les compositions dermatologiques ou cosmétiques telles que définies ci-dessus, caractérisées en ce que la source d'acide linoléique est une huile végétale ou un mélange de deux ou plusieurs huiles végétales, riches en acide linoléique ou un phospholipide riche en acide linoléique.

La présente invention a plus précisément pour objet les compositions dermatologiques ou cosmétiques telles que définies ci-dessus, caractérisées en ce que la source d'acide linoléique est une huile végétale ou un mélange de deux ou plusieurs huiles végétales riches en acide linoléique contenant au moins 20 % d'acide linoléique et de préférence 50 %.

Dans les compositions de l'invention, telles que définies ci-dessus, l'apport d'acide linoléique peut ainsi être réalisé en choisissant une huile contenant au moins 20 % de cet acide gras, comme l'huile de pépins de raisin, l'huile d'arachide, l'huile de noyaux d'abricot, l'huile d'amandes douces, l'huile de noisettes, l'huile de coton, l'huile de son de riz, l'huile de sésame, l'huile de noyau de cerises, l'huile de bourrache, l'huile de colza et de préférence une huile contenant plus de 50 % d'acide linoleique comme l'huile d'onagre, l'huile de tournesol, l'huile de carthame, l'huile de soja, l'huile de germe de blé, l'huile de fleur de la passion, l'huile d'oeillette, l'huile de lin. Comme source de l'acide linoléique, on préfère l'huile de germe de blé.

L'apport en acide linoléique peut être également fait en mélangeant deux ou plusieurs de ces huiles. On peut utiliser également une huile semi-synthétique : triglycéride, enrichie en acide linoléique. La teneur en huile riche en acide linoléique des préparations suivant l'invention peut varier de 1 à 20 % préférablement 2 à 10 %.

La teneur en acide linoléique acide des préparations suivant l'invention peut varier de 1 à 15 % préférablement de 1 à 10 %.

L'apport en acide linoléique peut également être fait avec des phospholipides d'origine végétale ou animale, particulièrement des lécithines d'oeuf et de soja, plus ou moins déhuilées, plus ou moins enrichies en phosphatridinyl choline, ou bien enrichies en phosphatidyl ethanolamine ou bien enrichies en phosphatidylinositol, un complexe de phospholipides issus de lécithine de soja déhuilée contenant 55 à 58 % d'acide linoléique.

La teneur en phospholipides riche en acide linoléique des préparations suivant l'invention peut varier de 1 à 30 %, préférablement de 2 à 10 %.

La présente invention a donc notamment pour objet les compositions dermatologiques ou cosmétiques, caractérisées en ce que la teneur en huile riche en acide linoléique peut varier de 1 à 20 % préférablement de 2 à 10 %, que la teneur en phospholipides riches en acide linoléique peut varier de 1 à 30 % préférablement de 2 à 10 %.

Les compositions selon l'invention peuvent si désiré, également contenir des principes actifs hydrosolubles ou liposolubles, des filtres, des écrans aux radiations solaires, des extraits vitaminés, des parfums, des conservateurs, des anti-oxydants, des colorants. Notamment les principes actifs hydrosolubles peuvent être hydratants, régénérants, anti-inflammatoires. Les principes actifs liposolubles peuvent être cicatrisants, nutritifs ou anti-phlogistiques.

Des filtres solaires et des réflecteurs solaires peuvent donc, également et si désiré, être ajoutés aux compositions cosmétiques et dermatologiques, selon l'invention telles que définies ci-dessus, pour leur conférer un pouvoir protecteur vis à vis des rayonnements solaires. Lorsque ces additifs sont insolubles dans les phases huileuses et aqueuses, ils constituent donc une phase supplémentaire. Ils sont choisis par exemple parmi les produits suivants :
- les perfluoroéthers comme les FOMBLIN (R) de la Société MONTECATINI,
- les pigments insolubles comme :
   . oxyde de titane,
   . oxyde de titane rutile,
   . oxyde de titane anatase,
   . oxyde de titane pyrogéné comme le P 25 (R) de Degussa,
   . oxyde de titane micronisé dans le SUN VEIL (R) d'Ikeda,
   . oxyde de titane traité en surface par des silicones ou par des acides aminés, ou par de la lécithine, ou par des stéarates métalliques,
   . oxyde de fer,
   . oxyde de fer traité en surface par des silicones, ou par des acides aminés, ou par de la lécithine, ou par des stéarates métalliques,
   . oxyde de zinc,
   . oxyde de zinc micronisé comme l'UFZO (R) de Cosmo Trends Corporation,
   . mica recouvert d'oxyde de titane.

La présente invention a ainsi pour objet des compositions dermatologiques ou cosmétiques telles que définies ci-dessus, caractérisées en ce que un ou plusieurs principes actifs hydrosolubles peuvent être incorporés dans la phase aqueuse de l'émulsion.

La présente invention a également pour objet des préparations dermatologiques ou cosmétiques telles que décrites ci-dessus, caractérisées en ce que un ou plusieurs principes actifs hydrosolubles sont choisis parmi le lactate de sodium (kératolytique et hydratant), des extraits de biolysat Hafnia (anti-inflammatoire et facteurs de croissance), des extraits de biolysat de Klebsiella pneumoniae (anti-élastase et anti-collagénase) et des filtres solaires hydrosolubles (protecteurs contre les rayonnements solaires UVA et UVB).

Ainsi, de façon préférentielle, les filtres solaires hydrosolubles et/ou les principes actifs hydrosolubles peuvent être choisis parmi :
- l'acide 2-phényl benzimidazol 5-sulfonique neutralisé,
- l'acide 2-hydroxy 4-méthoxybenzophénone 5-sulfonique neutralisé,
- l'acide ascorbique, le benzoate de caféine, l'acide phytique, l'acide mucique, les hydrolysats de protéines végétales, le polyglucan,
- l'extrait de mimosa du Mexique, le chitosan, le sérum d'animaux marins,
- l'extrait d'hirudine, l'extrait de méristem, les oligomères procyanodoliques, les extraits de levures, le panthénol, l'extrait de centella asiatica et l'acide glycyrrhétinique.

A titre d'illustration d'une telle utilisation de tels principes actifs hydrosolubles, mais sans toutefois limiter les quantités utilisables de ces principes actifs, les exemples suivants peuvent être donnés dans lesquels les pourcentages préférés sont exprimés par rapport à la formule complète finale :
- Acide 2-phényl benzimidazol 5-sulfonique neutralisé : 0,5 à 8 %,
- Acide 2-hydroxy 4-méthoxybenzophénone 5-sulfonique neutralisé : 0,5 à 5 %,
- Acide ascorbique : 0,5 à 10 %, benzoate de caféine : 0,1 à 5 %, acide phytique : 0,1 à 5 %, acide mucique : 0,1 à 5 %, hydrolysats de protéines végétales : 0,1 à 10 %, polyglucan : 0,1 à 5 %,
- Extrait de mimosa du Mexique : 0,5 à 20 %, chitosan : 0,5 à 20 %, sérum d'animaux marins : 0,1 à 3 %,
- Extrait d'hirudine : 0,5 à 10 %, extrait de méristem : 0,1 à 5 %, oligomères procyanodoliques : 0,05 à 3 %, extraits de levures : 0,05 à 3 %, panthénol : 0,05 à 5 %, extrait de centella asiatica : 0,05 à 3 %, l'acide glycyrrhétinique : 0,05 à 2 %.

La présente invention a également pour objet des préparations dermatologiques ou cosmétiques telles que décrites ci-dessus, caractérisées en ce que un ou plusieurs principes actifs liposolubles autres que les céramides et la source d'acide linoléique sont incorporés dans la phase grasse.

La présente invention a ainsi également pour objet des préparations dermatologiques ou cosmétiques, telles que décrites ci-dessus, caractérisées en ce que les principes actifs liposolubles sont choisis parmi le palmitate de vitamine A, des filtres solaires liposolubles, des insaponifiables d'origine végétale, de l'acétate de tocophérols, des tocophérols naturels, du farnesol.

De façon préférentielle mais sans toutefois limiter l'invention, on peut indiquer que :
- les filtres solaires liposolubles peuvent être choisis parmi l'octyl méthoxycinnamate, l'isoamyl méthoxycinnamate, l'octyl diméthyl paba, l'octyl salicylate, le butyl méthoxydibenzoyl méthane, le benzophénone 3, l'octyl triazone, le 4-polyéthoxy 5-aminobenzoate d'éthyle, l'isopropyle 4-dibenzoyl méthane,
- les insaponifiables de végétaux peuvent être choisis parmi des insaponifiables de maïs, de karité, de soja et d'avocat.
- le mélange huileux contenant de l'acide xyménique peut être du Xyménoil (R) qui renferme 50 % de cet acide.

A titre d'illustration d'une telle utilisation de tels principes actifs liposolubles, les exemples suivants peuvent être donnés dans lesquels les pourcentages préférés sont exprimés par rapport à la formule complète finale.
- Le palmitate de vitamine A : de 500 à 10 000 UI/g
- Des filtres solaires liposolubles : octyl méthoxycinnamate : 0,5 à 10 %, isoamyl éthoxycinnamate : 0,5 à 10 %, octyl diméthyl 25 paba : 0,5 à 8 %, octyl salicylate : 0,5 à 5 %, butyl méthoxy-dibenzoyl méthane : 0,5 à 5 %, benzophénone 3 : 0,5 à 10 %, octyl triazone : 0,5 à 5 %, 4-polyéthoxy aminobenzoate d'éthyle : 0,5 à 10 %, isopropyle 4-dibenzoyl méthane : 0,5 à 5 %,
- Des insaponifiables de maïs, de karité, de soja ou d'avocat : 0,1 à 3 %,
- Du Ximénoil (R) : 0,1 à 5 %, de l'extrait essentiel d'huile de sésame : 0,1 à 4 %, de l'huile de maïs péroxydée : 0,1 à 10 %, l'acétate de tocophérols : 0,05 à 7 %, des tocophérols naturels : 0,05 à 5 %, du farnesol : 0,05 à 5 %.

Les principes actifs de l'invention peuvent être incorporés à des excipients habituellement employés dans ces compositions dermatologiques ou cosmétiques.

Pour chaque forme, on a recours à des excipients appropriés. Ces excipients doivent avoir toutes les qualités habituellement demandées. Ils doivent être doués d'une grande affinité pour la peau, être parfaitement bien tolérés, stables, présenter une consistance adéquate permettant une utilisation facile et agréable.

A titre d'exemple d'excipients pouvant être utilisés, on peut citer notamment les excipients de phase grasse, les hydrocarbures comme le diisopropylcylohexane ou le polyisobutène hydrogéné, les huiles de silicone, les triglycérides naturels, les triglycérides semi-synthétiques ou synthétiques, les cires végétales telle que la cire de carnauba, animales telle que la cire d'abeille, ou minérales telle que l'ozokérite, les acides gras comme l'acide stéarique, les alcools gras comme l'alcool cétylique ou l'alcool isocétylique, les esters d'acide gras comme le palmitate d'octyle, les esters d'alcools gras, les amides d'acides gras, la lanoline et ses dérivés usuels tels que les alcools de lanoline, les acides de lanoline, la lanoline hydroxylée ou hydrogénée, les huiles minérales telle que l'huile de vaseline, des huiles végétales comme l'huile d'avocat ou de jojoba, des huiles végétales hydrogénées comme l'huile de palme hydrogénée.

On peut également citer les excipients de phase aqueuse comme les humectants tels la glycérine, le propylène glycol, le PEG400, le sorbitol. On peut citer les agents gélifiants comme les polymères carboxyvinyliques, la gomme Xanthane, la gomme Guar, les polymères carboxyvinyliques modifiés comme le PEMULEN(R), la Sclerosium gomme, les CMC, les CMC modifiées, les hydroxyéthylcelluloses, les hydroxypropylcelluloses, les hydroxyéthylcelluloses modifiées comme l'AMERCELL HM1500(R), les agents épaississants comme le VEEGUM(R).

Parmi les excipients, on utilise fréquemment des agents tensio-actifs comme des esters de sorbitan tel le stéarate de sorbitan, des esters de sorbitan oxyéthylénés comme le palmitate de sorbitan POE, des esters de sucrose comme le sucrose cocoate, des esters de glucose ou de méthylglucose oxyéthylénés ou non, comme le méthylgluceth-20 ou le méthylglucose sesquistéarate, des acyl phosphates neutralisés comme le potassium cétylphosphate, des acides gras éthoxylés comme l'acide stéarique éthoxylé, les alcools gras éthoxylés comme l'alcool stéarylique éthoxylé, les lécithines plus ou moins déhuilées, d'oeuf ou de soja, hydrogénées ou non, des stérols végétaux éthoxylés.

Les excipients peuvent également contenir des mouillants, des conservateurs tels le méthylparaben, le biosol, le bronopol, des parfums, des colorants, des charges comme le talc ou le polyméthacrylate.

Les différentes formes cosmétiques ou dermatologiques mentionnées ci-dessus peuvent être obtenues selon les méthodes usuelles utilisées dans ce domaine.

Les compositions dermatologiques et cosmétiques selon l'invention telles que définies ci-dessus, peuvent être par exemple, solides ou liquides ou pâteuses et se présentent sous les formes dermatologiques et cosmétiques couramment utilisées comme par exemple les crèmes ou gels en pots ou en tubes, les laits en flacons de verre ou de matière plastique et éventuellement en flacon-doseur, ampoule, les fluides, les pommades. Elles sont préparées selon les méthodes usuelles.

L'invention a particulièrement pour objet les compositions pharmaceutiques telles que définies ci-dessus, caractérisées en ce qu'elles se présentent sous forme de préparations liquides ou solides à usage topique et notamment les compositions pharmaceutiques telles que définies ci-dessus caractérisées en ce qu'elles se présentent préférentiellement sous une des formes suivantes :
- des gels gras,
- des émulsions simples, eau dans huile,
- des émulsions simples, huile dans eau,
- des émulsions multiples, par exemple :
- eau dans huile dans eau ou huile dans eau dans huile,
- une émulsion triple eau dans huile dans eau,
- une émulsion triple huile dans eau dans huile,
- une émulsion huile dans eau contenant des cristaux liquides,
- des émulsions complexes contenant des cristaux liquides formant des bicouches lipidiques entourant des phases grasses,
- des peudo-émulsions (dispersion d'une phase huileuse ou d'une émulsion eau dans huile dans une phase aqueuse gélifiée, sans tensio-actifs traditionnels),
- des micro-émulsions huile dans eau ou eau dans huile,
- des émulsions contenant des phases huiles dispersées, différentes et insolubles entre elles,
- une pseudo-émulsion ou dispersion d'une phase grasse dispersée dans une phase aqueuse et stabilisée avec du Lubragel (R), du Pemulen (R), de l'Hypan (R), de la gomme Xanthane, de la CMC, de l'hydroxyéthyl cellulose, de l'Amigel (R), de la Polyvinyl-pyrrolidone, de l'Amercell HM1500 (R), ou un mélange de deux ou plusieurs de ces gélifiants.

La présente invention a particulièrement pour objet l'utilisation de compositions dermatologiques ou cosmétiques, caractérisées en ce qu'elles contiennent des céramides et une source d'acide linoléique, pour restaurer la fonction barrière du stratum corneum des peaux sèches et très sèches.

La présente invention a plus particulièrement pour objet des compositions telles que définies ci-dessus, caractérisées en ce qu'elles se présentent sous forme de :
- émulsions simples, eau dans huile,
- émulsions simples, huile dans eau,
- émulsion triple eau dans huile dans eau,
- émulsions complexes contenant des cristaux liquides formant des bicouches lipidiques entourant des phases grasses,
- peudo-émulsions (dispersion d'une phase huileuse ou d'une émulsion eau dans huile dans une phase aqueuse gélifiée, sans tensio-actifs traditionnels),
- pseudo-émulsion ou dispersion d'une phase grasse dispersée dans une phase aqueuse et stabilisée avec du Lubragel (R), du Pemulen (R), de l'Hypan (R), de la gomme Xanthane, de la CMC, de l'hydroxyéthyl cellulose, de l'Amigel (R), de la Polyvinyl-pyrrolidone, de l'Amercell HM1500 (R), ou un mélange de deux ou plusieurs de ces gélifiants.

La présente invention a notamment pour objet l'application à titre de produit cosmétique des compositions caractérisées en ce qu'elles contiennent des céramides et une source d'acide linoléique.

La présente invention a tout particulièrement pour objet une méthode de traitement cosmétique de restauration de la fonction barrière du stratum corneum des peaux sèches et très sèches, caractérisée en ce que l'on applique sur la peau une quantité suffisante d'une composition cosmétique telle que définie ci-dessus.

On démontre dans les exemples suivants, la supériorité de cette association céramides-acide linoléique sur les éléments séparés.

### EXEMPLE 1 : Crème I

On chauffe à 70°C la phase grasse suivante :

| | |
|---|---|
| - Stearamidopropyl PG-dimonium chloride Phosphate (nom CTFA) | 3,0 g |
| - Cocamidopropyl PG-dimonium chloride Phosphate (nom CTFA) | 1,0 g |
| - Alcool cétylique | 3,0 g |
| - Myristyl myristate | 5,0 g |
| - Polyisobutene hydrogéné | 2,0 g |
| - Beurre de karité | 2,0 g |
| - Stéarate de Propylène glycol | 3,0 g |
| - Huile de silicone | 2,0 g |
| - Huile de germe de blé contenant au moins 50 % d'acide linoléique | 5,0 g |
| - Anti-oxydant | 0,2 g |
| - Céramide HO3 (SEDERMA) | 0,2 g |

On prépare par ailleurs la phase aqueuse suivante et on la chauffe à 70°C. :

| | |
|---|---|
| - Eau déminéralisée | 61,08 g |
| - Glycérine | 10,0 g |
| - Hydroxy éthylcellulose modifiée | 0,5 g |
| - PVP | 1,0 g |
| - Conservateurs | 0,52 g |

On réalise l'émulsion H/E en mélangeant vivement les deux phases à 90°C pendant 10 minutes. On refroidit ensuite lentement sous agitation modérée. A 45°C, on ajoute 0,5 % de parfum, puis on continue à refroidir jusqu'à 25°C.

On obtient ainsi une émulsion H/E cationique dénommée crème I de couleur blanc ivoire, agréable au toucher, ph 5,6 et de viscosité Brookfield 24 000 cps.

### TEST D'HYDRATATION

On procède au test d'hydratation décrit ci-après afin de comparer l'activité d'une part, de la crème I selon l'invention, préparée ainsi qu'il est indiqué à l'exemple 1 qui renferme des céramides et de l'acide linoléique avec les activités respectives de deux crèmes II et III telles que la crème II renferme des céramides mais pas d'acide linoléique et la crème III renferme de l'acide linoléique, mais pas de céramides.

Les résultats qui suivent obtenus par le test d'hydratation, montrent ainsi la supériorité de la crème I sur les crèmes II et III.

Les crèmes II et III sont préparées comme suit :

### Crème II

On procède comme à l'exemple 1, en remplaçant l'huile de germe de blé par un triglycéride saturé caprylique/caprique. On chauffe à 70°C la phase grasse suivante :

| | |
|---|---|
| - Stearamidopropyl PG-dimonium chloride Phosphate (nom CTFA) | 3,0 g |
| - Cocamidopropyl PG-dimonium chloride Phosphate (nom CTFA) | 1,0 g |
| - Alcool cétylique | 3,0 g |
| - Myristyl myristate | 5,0 g |
| - Polyisobutene hydrogéné | 2,0 g |
| - Beurre de karité | 2,0 g |
| - Stéarate de Propylene glycol | 3,0 g |
| - Huile de silicone | 2,0 g |
| - Triglycéride saturé caprylique/caprique | 5,0 g |
| - Anti-oxydant | 0,2 g |
| - Céramide HO3 (SEDERMA) | 0,2 g |

On prépare par ailleurs la phase aqueuse suivante et on la chauffe à 70°C. :

| | |
|---|---|
| - Eau déminéralisée | 61,08 g |
| - Glycérine | 10,0 g |
| - Hydroxy éthylcellulose modifiée | 0,5 g |
| - PVP | 1,0 g |
| - Conservateurs | 0,52 g |

Comme à l'exemple 1, on réalise l'émulsion H/E en mélangeant vivement les deux phases à 90°C pendant 10 minutes. On refroidit ensuite lentement sous agitation modérée. A 45°C, on ajoute 0,5 % de parfum, puis on continue à refroidir jusqu'à 25°C.

On obtient ainsi une émulsion H/E cationique dénommée crème II. La crème II ainsi obtenue renferme donc des céramides mais pas d'acide linoléique.

### Crème III

On procède comme à l'exemple 1 et chauffe à 70°C la phase grasse suivante :

| | |
|---|---|
| - Stearamidopropyl PG-dimonium chloride Phosphate (nom CTFA) | 3,0 g |
| - Cocamidopropyl PG-dimonium chloride Phosphate (nom CTFA) | 1,0 g |
| - Alcool cétylique | 3,0 g |
| - Myristyl myristate | 5,0 g |
| - Polyisobutene hydrogéné | 2,0 g |
| - Beurre de karité | 2,0 g |
| - Stéarate de Propylene glyco | 3,0 g |
| - Huile de silicone | 2,0 g |
| - Huile de germe de blé contenant au moins 50 % d'acide linoléique | 5,0 g |
| - Anti-oxydant | 0,2 g |

On prépare par ailleurs la phase aqueuse suivante et on la chauffe à 70°C. :

| | |
|---|---|
| - Eau déminéralisée | 61,08 g |
| - Glycérine | 10,0 g |
| - Hydroxy éthylcellulose modifiée | 0,5 g |
| - PVP | 1,0 g |
| - Conservateurs | 0,52 g |

Comme à l'exemple 1, on réalise l'émulsion H/E en mélangeant vivement les deux phases à 90°C pendant 10 minutes. On refroidit ensuite lentement sous agitation modérée. A 45°C, on ajoute 0,5 % de parfum, puis on continue à refroidir jusqu'à 25°C.

On obtient ainsi une émulsion H/E cationique dénommée crème III. La crème III ainsi obtenue renferme donc de l'acide linoléique mais pas de céramides.

### PROTOCOLE DU TEST D'HYDRATATION

### 1. PARTICIPANTS

Une Dermatologue a sélectionné 4 adultes, 3 femmes, 1 homme, de race caucasienne, sains, âgés de 25 à 50 ans. Ces participants s'engagent à suivre leur usage habituel en matière de toilette, mais s'abstiendront de toute application de produit de soin cosmétique sur les bras.

### 2. PROTOCOLE D'APPLICATION

Zone de mesure et d'application :
Avant-bras, intérieur ou extérieur zone de 10 cm²
Zone A (la plus haute) Bras droit, CREME II
Zone B (au milieu) Bras droit, CREME I
Zone C (en bas) Bras droit, sans traitement (zone témoin)
Zone D (la plus haute) Bras gauche, CREME III
Zone E (au milieu) Bras gauche, CREME I
Zone F (en bas) Bras gauche, sans traitement (zone témoin)

### Dose d'application :

2 mg/cm² soit 20 mg/10 cm²

### Protocole d'application :

Sur une peau propre, avec un massage de quelques secondes à l'aide d'un doigt recouvert par un doigtier de caoutchouc.

### Périodicité d'application :

5 applications : en 8 jours maximum, 1 fois par jour

### 3. MESURES

Le degré d'hydratation de la couche cornée au stratum corneum sera déterminé à l'aide d'un cornéomètre.

Dans chaque zone, 3 mesures seront réalisées et la moyenne sera calculée.

Le principe de l'appareil est basé sur la mesure des propriétés diélectriques de la peau. La lecture se fait directement sur l'appareil qui mesure à l'aide d'un condensateur la capacité électrique d'une portion de peau placée sous la sonde.

Une mesure sera réalisée avant traitement, puis après la première application (au moins 10 minutes après, au plus 30 minutes après). Les autres mesures auront lieu les autres jours à peu près à la même heure, avant les nouvelles applications quand il y a lieu.

Les mesures se poursuivront tous les jours ouvrables pendant 10 jours environ après la dernière application.

On suivra les différences obtenues par application des crèmes I, II et III sur chaque bras, la 3ème zone servant de temoin pour vérifier l'évolution propre de la peau.

On suivra ces différences pendant la période où le produit est appliqué et après arrêt du traitement.

A chaque mesure, on notera l'heure, la température ambiante et le degré hygrométrique de la pièce où sont effectuées les mesures.

### 4. RESULTATS

Ils sont consignés sur des feuilles, une par participant. Chaque feuille reprend toutes les indications relatives aux mesures : date, heure, température de la pièce et degré hygrométrique, les 3 mesures effectuées sur chaque zone et la moyenne de ces 3 mesures.

### PONDERATION

Chaque moyenne de chaque zone est affectée de 2 pondérations : une variation en pourcentage, en plus ou en moins, qui tient compte de l'écart de mesure constaté à J0 avant application entre les zones destinées à l'application et les zones témoins du même bras.

Cette pondération s'applique sur toutes les mesures d'une même zone.

Une deuxième pondération est effectuée pour les mesures d'un jour donné en tenant compte de la différence constatée entre la mesure de la zone témoin ce jour là et celle du J0.

Cela revient à dire que les variations entre zones et les variations propres de la peau de chaque sujet sont égalisées.

### RESULTATS

Les résultats pondérés servent à calculer les différences d'hydratation produites par la crème I selon l'invention qui renferme des céramides et de l'acide linoléique, avec d'une part la crème II qui renferme des céramides mais pas d'acide linoléique, et d'autre part la crème III qui renferme de l'acide linoléique, mais pas de céramides.

Les moyennes arithmétiques de ces différences sont calculées.

Les moyennes du J11 du sujet AF sont intégrées avec les moyennes des J13 des autres sujets.

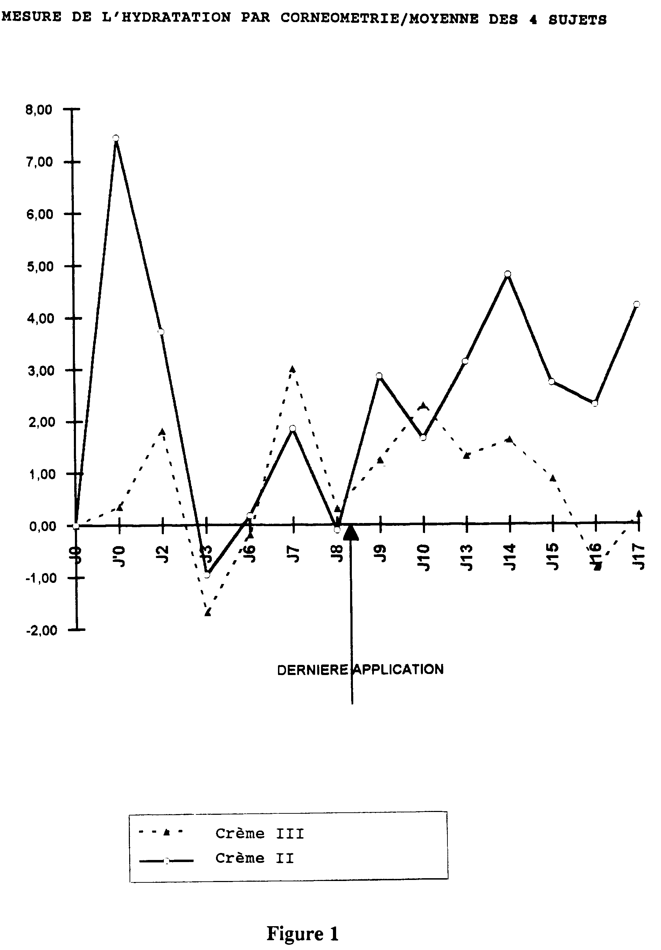

Les tableaux Ia et Ib ci-après qui indiquent les résultats obtenus par le test d'hydratation décrit ci-dessus montrent donc que les moyennes des écarts sont presque toujours positives, notamment après arrêt des applications : les tableaux Ia et Ib ainsi que la figure I ci-après qui illustre les résultats, montrent donc que l'association céramides et acide linoléique dans une crème hydratante produit un effet hydratant supérieur à celui des crèmes qui n'ont qu'un seul de ces ingrédients.

Les résultats indiqués dans les tableaux Ia et Ib, montrent notamment que, au moins jusqu'à 10 jours après la dernière application faite le 8^{e} jour (J8), l'hydratation produite par l'application de la crème I est toujours supérieure (moyenne >0) à celle produite par l'application des crèmes II et III.

Dans les tableaux Ia et Ib, on voit que la colonne "moyenne" est presque toujours positive, ce qui signifie que le taux d'hydration à l'endroit traité par la crème complète est supérieur au taux d'hydration des crèmes incomplètes, ce qui démontre l'avantage d'associer dans une crème hydratante des céramides et une source d'acide linoléique.

**TABLEAU Ia**

| **Compilation des résultats pondérés** | | | | | |
|---|---|---|---|---|---|
| | Sujets traités par les crèmes I et II | | | | MOYENNE |
| N^{·} J | 1 | 2 | 3 | 4 | |
| JO | 0,00 | 0,00 | 0,00 | 0,00 | 0,00 |
| J'0 | 17,83 | -8,23 | -1,46 | -6,71 | 0,36 |
| J2 | 6,63 | | | -3,00 | 1,82 |
| J3 | -6,40 | 4,05 | -1,13 | -3,22 | -1,68 |
| J6 | | | -0,37 | 0,02 | -0,18 |
| J7 | 2,38 | 7,42 | -0,77 | | 0,32 |
| J8 | | 1,59 | 1,20 | -1,84 | 0,32 |
| J9 | 5,61 | -0,41 | 0,96 | -1,15 | 1,25 |
| J10 | 7,31 | 1,60 | 0,91 | -0,63 | 2,30 |
| J13 | 2,22 | 3,04 | 1,38 | -1,37 | 1,32 |
| J14 | 6,22 | 2,05 | -1,66 | -0,10 | 1,63 |
| J15 | 2,83 | 0,78 | 2,82 | 2,92 | 0,88 |
| J16 | 4,12 | 1,53 | -3,52 | -5,52 | -0,85 |
| J17 | 3,16 | 0,17 | -3,86 | 1,31 | 0,20 |
| TOTAL | 51,91 | 13,59 | -5,50 | -25,13 | 0,80 |

**TABLEAU Ib**

| **Compilation des résultats pondérés** | | | | | |
|---|---|---|---|---|---|
| | Sujets traités par les crèmes I et III | | | | MOYENNE |
| N^{·} J | 1 | 2 | 3 | 4 | |
| JO | 0,00 | 0,00 | 0,00 | 0,00 | 0,00 |
| J'0 | 6,16 | 0,65 | 9,95 | 13,00 | 7,44 |
| J2 | 9,56 | | | -2,10 | 3,73 |
| J3 | 4,12 | -5,63 | 1,53 | -3,86 | -0,96 |
| J6 | | | 0,42 | -0,06 | 0,18 |
| J7 | 8,15 | -3,55 | 0,95 | | 1,85 |
| J8 | | -1,10 | 0 41 | 0,38 | -0,10 |
| J9 | 16,29 | -0,61 | -2,45 | -1,80 | 2,86 |
| J10 | 9,31 | 2,45 | -3,06 | -2,06 | 1,66 |
| J13 | 6,41 | 7,27 | 1,82 | -3,00 | 3,13 |
| J14 | 10,10 | 6,01 | 3,67 | -0,57 | 4,80 |
| J15 | 9,57 | 4,57 | 1,81 | -5,03 | 2,73 |
| J16 | 3,04 | 6,72 | 1,41 | -2,00 | 2,29 |
| J17 | 5,45 | 2,74 | 2,29 | 6,31 | 4,20 |
| TOTAL | 88,16 | 19,52 | 18,75 | -0,79 | 2,60 |

### EXEMPLE 2 : Crème pour le visage

On prépare comme à l'exemple 1, la crème constituée de :

| | |
|---|---|
| - Céramide | 0,5 g |
| - Huile d'onagre | 2,0 g |
| - Alcoyle phosphate de potassium | 2,0 g |
| - Palmitate d'éthyl hexyle | 8,0 g |
| - Lanoline hydrogénée | 5,0 g |
| - Triglycérides d'acides gras | 4,0 g |
| - Stéarate de sorbitan | 1,0 g |
| - Polymère carboxyvinylique neutralisé | 0,4 g |
| - Conservateurs | 0,4 g |
| - Composition aromatique | 0,4 g |
| - Eau purifiée QSP | 100,0 g |

### EXEMPLE 3 : Crème pour le corps

On prépare comme à l'exemple 1, la crème constituée de :

| | |
|---|---|
| - Céramide de blé | 0,2 g |
| - Stéarate de glycérol | 4,0 g |
| - Palmitate de sorbitan | 6,0 g |
| - Perhydrosqualène | 5,0 g |
| - Diisopropyle-cyclohexane | 7,0 g |
| - Huile de tournesol | 9,0 g |
| - Glycérine | 5,0 g |
| - Conservateurs | 0,35 g |
| - Composition aromatique | 1,0 g |
| - Eau purifiée QSP | 100,0 g |

### EXEMPLE 4 : Lait solaire

On prépare comme à l'exemple 1, la crème constituée de :

| | |
|---|---|
| - Céramide de lait | 0,1 g |
| - Filtres solaires | 5,0 g |
| - Huile de vaseline | 10,0 g |
| - Cétéaryl octanoate | 4,0 g |
| - Phospholipides de soja déhuilé | 5,0 g |
| - Huile de silicone | 2,5 g |
| - Ether cétylique P.O.E. | 2,0 g |
| - Stéarate de sorbitan | 1,0 g |
| - Conservateurs | 0,35 g |
| - Composition aromatique | 0,5 g |
| - Eau purifiée QSP | 100,0 g |

### EXEMPLE 5 : Emulsion multiple

On chauffe à 80°C la phase aqueuse suivante, dite phase aqueuse interne :

| | |
|---|---|
| - Eau déminéralisée | 26,52 g |
| - Méthylparaben | 0,1 g |
| - Sulfate de magnésium | 0,28 g |
| - Glycérine 30' B | 0,8 g |
| - O-cymen-5-ol | 0,04 g |

On chauffe séparément la phase grasse suivante :

| | |
|---|---|
| - Glycéryl isostéarate | 2 g |
| - Huile de ricin hydrogénée polyoxyéthylénée (7 moles) | 0,2 g |
| - Huile de soja | 8,2 g |
| - Propylparaben | 0,06 g |
| - Huile de silicone volatile | 1,6 g |
| - Céramide HO3 | 0,5 g |

On disperse la phase aqueuse dans la phase grasse à 80°C en agitant vigoureusement 5 minutes. Puis on refroidit lentement jusqu'à 25°C.

On disperse ensuite cette émulsion primaire eau/huile dans la phase aqueuse suivante, dite phase aqueuse externe, en mélangeant doucement à température ambiante :

| | |
|---|---|
| - Eau déminéralisée QSP | 100,0 g |
| - Lubragel MS (R) | 15,0 g |
| - Carbopol 980 (R) | 0,03 g |
| - Tétrasodium EDTA | 0,054 g |
| - Méthylparaben | 0,216 g |
| - Imidazolidinyl urée | 0,216 g |
| - Hydroxyde de sodium pur | 0,125 g |

### EXEMPLE 6 : Emulsion à phases gémellaires

On fait chauffer la phase grasse suivante à 80°C :

| | |
|---|---|
| - Alcool stéarylique | 1,0 g |
| - Alcool cétylique | 2,0 g |
| - Cétéaryl octanoate | 4,0 g |
| - Polysorbate 60 | 4,0 g |
| - Sorbitan stéarate | 4,0 g |
| - Huile de carthame | 6,0 g |
| - Beurre de karité | 3,0 g |
| - Acétate d'oleyle | 2,0 g |
| - Huile de silicone | 0,5 g |
| - Tocophérols | 0,05 g |
| - Céramide HO3 | 0,5 g |

On fait chauffer la phase aqueuse suivante à 80°C :

| | |
|---|---|
| - Eau déminéralisée QSP | 100,0 g |
| - Polymère carboxy vinylique | 0,3 g |
| - Conservateur | 0,7 g |
| - Lubragel MS (R) | 5,0 g |
| - Hydroxyde de sodium pur | 0,3 g |

On disperse la phase grasse dans la phase aqueuse et on agite vigoureusement pendant 10 minutes. On refroidit ensuite lentement l'émulsion ainsi formée jusqu'à 25°C, on y ajoute alors le parfum sous agitation modérée :

| | |
|---|---|
| - Parfum | 0,2 g |

### EXEMPLE 7 : Emulsion eau/silicone

On chauffe à 60°C la phase grasse suivante :

| | |
|---|---|
| - Eau déminéralisée QSP | 100,0 g |
| - Chlorure de sodium | 0,8 g |
| - Acide citrique pur | 0,01 g |
| - Méthylparaben | 0,25 g |
| - Propylène glycol | 2,0 g |
| - O-cymen-5-ol | 0,1 g |

On chauffe à 60°C la phase silicone suivante :

| | |
|---|---|
| - Stéarate d'isocétyle | 3,0 g |
| - Arlacel 83 (R) | 0,8 g |
| - Huile de ricin hydrogénée | 0,3 g |
| - Elfacos ST9 (R) | 2,0 g |
| - Acétate d'oleyle (anti-lipase) | 0,15 g |
| - Silicone DC 3225 (R) (DOW CORNING) | 9,0 g |
| - Silicone volatile | 4,0 g |
| - Céramide de blé | 0,5 g |
| - Huile d'oeillette | 3,0 g |

On disperse la phase aqueuse dans la phase silicone sous agitation moyenne pendant 10 minutes. On refroidit ensuite l'émulsion ainsi formée jusqu'à 25°C et on ajoute alors le parfum :

| | |
|---|---|
| - Parfum | 0,3 g |

### EXEMPLE 8 : Emulsion sans émulsionnant

On chauffe à 80°C la phase grasse suivante :

| | |
|---|---|
| - Huile de germe de blé | 4,0 g |
| - Polyisobutène | 4,0 g |
| - Octyl stéarate | 4,0 g |
| - Céramide HO3 | 2,0 g |

On chauffe à 80°C la phase aqueuse suivante :

| | |
|---|---|
| - Glycérine 30' codex | 3,0 g |
| - Polymère carboxyvinylique | 0,45 g |
| - Lubragel MS (R) | 4,0 g |
| - Hydroxyde de sodium pur | 0,055 g |
| - Conservateurs | 0,55 g |
| - Parfum | 0,20 g |
| - Eau déminéralisée | 60,0 g |

On disperse la phase grasse dans la phase aqueuse sous une très faible agitation et cisaillement élevé pendant 1/2 heure. On refroidit ensuite lentement l'émulsion ainsi formée jusqu'à 45°C, on ajoute alors sous bonne agitation :

| | |
|---|---|
| - Talc | 3,0 g |

Quand la dispersion du talc est bien terminée, on continue à refroidir sous agitation lente. Quand la température est de 25°C, on ajoute sous agitation modérée :

| | |
|---|---|
| - Parfum | 0,2 g |

### EXEMPLE 9 :

On prépare une émulsion huile dans eau de la manière suivante.

On chauffe à 80°C les composants de la phase grasse suivante :

| | |
|---|---|
| - Stéarate de glycérol autoémulsionnable (arlacel 165 (R) de la Sté ICI) | 6,0 g |
| - Alcool cétylique | 1,0 g |
| - Stérol de soja éthoxylé (générol 122 E 10 (R) de la Sté Henkel) | 2,0 g |
| - Mélange d'huile de vaseline et d'alcool de lanoline (amerchol L101 (R) de la Sté Amerchol) | 3,0 g |
| - Pétrolatum et alcool de lanoline (amerchol CAB (R) de la Sté Amerchol) | 1,0 g |
| - Huile de carthame | 6,0 g |
| - Beurre de karité | 3,0 g |
| - Propyl paraben | 0,05 g |
| - Céramide HO3 | 0,5 g |

Par ailleurs on prépare la phase aqueuse suivante que l'on porte également à 80°C :

| | |
|---|---|
| - Eau déminéralisée | 60,0 g |
| - Sorbitol à 70 % | 3,0 g |
| - Gomme xanthane | 0,3 g |
| - Méthylparaben | 0,1 g |

Quand la gomme xanthane est bien dispersée, on ajoute la phase grasse à la phase aqueuse à 80°C, et on agite vivement pendant 20 minutes. L'émulsion se forme.

Ensuite on réduit l'agitation et on refroidit lentement l'émulsion jusqu'à 40°C.

On y ajoute alors 2 g d'eau contenant 0,15 g d'imidazolidinyl urée, puis 0,3 g de parfum.

## Revendications

1. Compositions dermatologiques ou cosmétiques pour les soins de la peau, caractérisées en ce qu'elles contiennent un céramide de formule (S) : 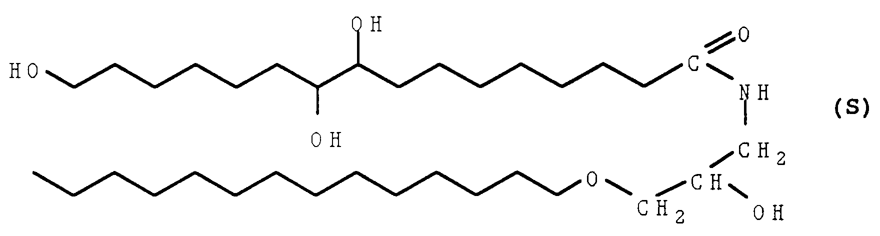 et une source d'acide linoléique, qui est une huile végétale riche en acide linoléique.

2. Compositions dermatologiques ou cosmétiques telles que définies à la revendication 1, caractérisées en ce que la teneur en céramides est de 0,1 à 15 %.

3. Compositions dermatologiques ou cosmétiques telles que définies aux revendications 1 et 2, caractérisées en ce que la teneur en céramides est de 0,1 à 1 %.

4. Compositions dermatologiques ou cosmétiques telles que définies aux revendications 1 à 3 caractérisées en ce que la source d'acide linoléique est une huile végétale contenant au moins 50 % d'acide linoléique.

5. Compositions dermatologiques ou cosmétiques telles que définies aux revendications 1 à 4, caractérisées en ce que la teneur en huile riche en acide linoléique peut varier de 2 à 10 %.

6. Compositions telles que définies aux revendications 1 à 5, caractérisées en ce qu'elles se présentent sous forme de :
- des émulsions simples, eau dans huile,
- des émulsions simples, huile dans eau,
- une émulsion triple eau dans huile dans eau,
- des émulsions complexes contenant des cristaux liquides formant des bicouches lipidiques entourant des phases grasses,
- des peudo-émulsions (dispersion d'une phase huileuse ou d'une émulsion eau dans huile dans une phase aqueuse gélifiée, sans tensio-actifs traditionnels),
- une pseudo-émulsion ou dispersion d'une phase grasse dispersée dans une phase aqueuse et stabilisée avec du Lubragel (R), du Pemulen (R), de l'Hypan (R), de la gomme Xanthane, de la CMC, de l'hydroxyéthyl cellulose, de l'Amigel (R), de la Polyvinyl-pyrrolidone, de l'Amercell HM1500 (R), ou un mélange de deux ou plusieurs de ces gélifiants.

7. Application à titre de produit cosmétique des compositions selon les revendications 1 à 5.

8. Méthode de traitement cosmétique de restauration de la fonction barrière du stratum corneum des peaux sèches et très sèches, caractérisée en ce que l'on applique sur la peau une quantité suffisante d'une composition cosmétique telle que définie selon l'une quelconque des revendications 1 à 5.

## Claims

1. Dermatological or cosmetic compositions for skin care, characterized in that they contain a ceramide of formula (S): 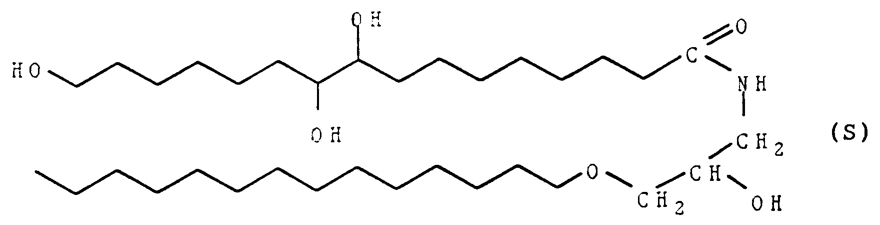 and a linoleic acid source, which is a vegetable oil rich in linoleic acid.

2. Dermatological or cosmetic compositions as defined in claim 1, characterized in that the ceramide content is 0.01 to 15%.

3. Dermatological or cosmetic compositions as defined in claims 1 and 2, characterized in that the ceramide content is 0.1 to 1%.

4. Dermatological or cosmetic compositions as defined in claims 1 to 3, characterized in that the linoleic acid source is a vegetable oil containing at least 50% linoleic acid.

5. Dermatological or cosmetic compositions as defined in claims 1 to 4, characterized in that the content of oil rich in linoleic acid can vary from 2 to 10%.

6. Compositions as defined in claims 1 to 5, characterized in that they are presented in the form of:
- simple water-in-oil emulsions,
- simple oil-in-water emulsions,
- a triple water-in-oil-in-water emulsion,
- complex emulsions containing liquid crystals forming lipidic double layers surrounding the oily phases,
- pseudo-emulsions (dispersion of an oily phase or a water-in-oil emulsion in a gelatinized aqueous phase, without traditional surfactants),
- a pseudo-emulsion or dispersion of an oily phase dispersed in an aqueous phase and stabilized with Lubragel (R), Pemulen (R), Hypan (R), xanthan gum, CMC, hydroxyethyl cellulose, Amigel (R), Polyvinyl-pyrrolidone, Amercell HM1500 (R), or a mixture of two or more of these gelatinizing agents.

7. Use as a cosmetic product of the compositions according to claims 1 to 5.

8. Cosmetic treatment method for restoring the barrier function of the stratum corneum of dry and very dry skin, characterized in that a sufficient quantity of a cosmetic composition as defined according to any one of claims 1 to 5 is applied to the skin.

## Patentansprüche

1. Dermatologische oder kosmetische Zusammensetzungen für die Pflege der Haut, dadurch **gekennzeichnet,** daß sie ein Ceramid der Formel (S) 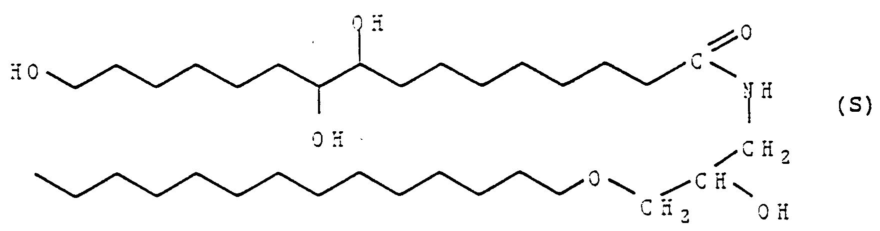 und eine Quelle für Linolsäure enthalten, die ein an Linolsäure reiches Pflanzenöl ist.

2. Dermatologische oder kosmetische Zusammensetzungen nach Anspruch 1, dadurch **gekennzeichnet,** daß der Gehalt an Ceramiden 0,1 bis 15% beträgt.

3. Dermatologische oder kosmetische Zusammensetzungen nach den Ansprüchen 1 und 2, dadurch **gekennzeichnet,** daß der Gehalt an Ceramiden 0,1 bis 1% beträgt.

4. Dermatologische oder kosmetische Zusammensetzungen nach den Ansprüchen 1 bis 3, dadurch **gekennzeichnet,** daß die Quelle für Linolsäure ein Pflanzenöl, das mindestens 50% Linolsäure enthält, ist.

5. Dermatologische oder kosmetische Zusammensetzungen nach den Ansprüchen 1 bis 4, dadurch **gekennzeichnet,** daß der Gehalt an an Linolsäure reichem Öl von 2 bis 10% variieren kann.

6. Zusammensetzungen nach den Ansprüchen 1 bis 5, dadurch **gekennzeichnet,** daß sie in Form von
- einfachen Emulsionen, Wasser in Öl,
- einfachen Emulsionen, Öl in Wasser,
- einer Dreifachemulsion Wasser in Öl in Wasser,
- komplexen Emulsionen, enthaltend Flüssigkristalle, die Lipiddoppelschichten bilden, die die fetten Phasen umgeben,
- Pseudoemulsionen (Dispersion einer Ölphase oder einer Wasser-in-Öl-Emulsion in einer wäßrigen gelierten Phase ohne übliche grenzflächenaktive Mittel),
- einer Pseudoemulsion oder Dispersion einer Fettphase, die in einer wäßrigen Phase dispergiert ist und mit Lubragel (R), Pemulen (R), Hypan (R), Xanthangummi, CMC, Hydroxyethylcellulose, Amigel (R), Polyvinylpyrrolidon, Amercell HM1500 (R) oder einem Gemisch dieser oder mehrerer dieser Gelbildner stabilisiert ist,
vorliegen.

7. Verwendung der Zusammensetzungen nach den Ansprüchen 1 bis 5 als kosmetisches Produkt.

8. Verfahren zur kosmetischen Behandlung zur Wiederherstellung der Barrierefunktion des Stratum corneum von trockener und sehr trockener Haut, dadurch **gekennzeichnet,** daß man auf die Haut eine ausreichende Menge einer kosmetischen Zusammensetzung nach einem der Ansprüche 1 bis 5 aufbringt.
